# EUROPEAN PATENT APPLICATION

(11) **EP 4 067 328 A1**
(43) Date of publication of application: **05.10.2022**
(21) Application number: 20892561.0
(22) Date of filing: 28.10.2020
(51) Int. Cl.: C07C 29/132, C07C 33/26, C07B 61/00

(54) **METHOD FOR PRODUCING AROMATIC ALCOHOLS**

(30) Priority: 26.11.2019 JP 2019213486
(71) Applicant: SUMITOMO CHEMICAL COMPANY, LIMITED, Tokyo 103-6020 (JP)
(72) Inventor: KOIKE, Hirofumi, Sodegaura-shi, Chiba 299-0246 (JP); SAWANO, Naoto, SINGAPORE LTD., 100, Ayer Merbau Road 628277 (SG); YOSHII, Masayuki, Ichihara-shi, Chiba 299-0195 (JP)
(74) Representative: Winter, Brandl - Partnerschaft mbB
(86) International application number: PCT/JP2020/040450
(87) International publication number: WO 2021/106480

(57) **Abstract**

An object is to provide a method for producing aromatic alcohols, the method enabling an improvement in production efficiency by reducing clogging of a filtering device for separating an alumina-supported transition metal catalyst from a reaction liquid when a hydrogenation reaction of aromatic peroxides is carried out in the presence of the alumina-supported transition metal catalyst, in a device including the filtering device. The object is achieved by including an aqueous cleaning step of cleaning a raw material composition containing aromatic peroxides, an alkali metal, and an organic solvent.

## Description

### Technical Field

The present invention relates to a method for producing aromatic alcohols.

### Background Art

There is a known method for producing bis(2-hydroxy-2-propyl)benzene by subjecting a raw material liquid which is a methyl isobutyl ketone solution containing, as a main component, (hydroperoxyisopropyl)(hydroxyisopropyl)benzene to a liquid phase hydrogenation reaction in the presence of a transition metal catalyst (see Patent Literature 1). Patent Literature 1 discloses a bis(2-hydroxy-2-propyl)benzene production method in which a hydrogenation reaction is carried out under a predetermined condition for the purpose of maintaining activity of a palladium catalyst for a long period of time.

### Citation List

### [Patent Literature]

[Patent Literature 1]
Japanese Patent Application Publication, Tokukaihei, No. 9-194411

### Summary of Invention

### Technical Problem

However, the conventional technique described above causes the following problem: In a case where a hydrogenation reaction of aromatic peroxides is carried out in the presence of an alumina-supported transition metal catalyst, in a device including a filtering device for separating the alumina-supported transition metal catalyst from a reaction liquid, the filtering device gets clogged as reaction time passes. Clogging of the filtering device leads to an increase in a differential pressure and thus makes it difficult to extract the reaction liquid. This requires stopping the plant to eliminate the clogging, and thus reduces production efficiency.

An object of an aspect of the present invention is to provide a method for producing aromatic alcohols, the method enabling an improvement in production efficiency by reducing the clogging of a filtering device for separating an alumina-supported transition metal from a reaction liquid, in a case where a hydrogenation reaction of the aromatic peroxides is carried out in the presence of the alumina-supported transition metal catalyst in a device including the filtering device.

### Solution to Problem

For achievement of the above object, a method, in accordance with an embodiment of the present invention, for producing aromatic alcohols includes: an aqueous cleaning step of cleaning, with water, a composition containing aromatic peroxides, an alkali metal, and an organic solvent; and a hydrogenation step of hydrogenating the aromatic peroxides contained in the composition having been cleaned with water in the aqueous cleaning step, in the presence of an alumina-supported transition metal catalyst, in a device including a filtering device for separating the alumina-supported transition metal catalyst from a reaction liquid.

### Advantageous Effects of Invention

A method, in accordance with an embodiment of the present invention, for producing aromatic alcohols has an effect of enabling an improvement in production efficiency by reducing the clogging of a filtering device for separating an alumina-supported transition metal catalyst from a reaction liquid, in a case where a hydrogenation reaction of the aromatic peroxides is carried out in the presence of the alumina-supported transition metal catalyst in a device including the filtering device.

### Description of Embodiments

The following description will discuss embodiments of the present invention in detail. In the present application, the expression "A to B" refers to "not less than A and not more than B".

The inventors of the present invention have diligently studied in view of the above object, and eventually found that the clogging of the filtering device results from the alumina-supported transition metal catalyst. Further, the alkali metal contained in the solution of the aromatic peroxides in the organic solvent, which is a raw material liquid, causes the clogging of the filtering device. In consideration of the above findings, the solution (which is a raw material liquid), containing the alkali metal, of the aromatic peroxides in the organic solvent (in other words, a composition containing the aromatic peroxides, the alkali metal, and the organic solvent) was cleaned with water, and a hydrogenation reaction was then carried out in the presence of the alumina-supported transition metal catalyst. The inventors found that this method enables a reduction in clogging of the filtering device, and accomplished the present invention.

A method, in accordance with an embodiment of the present invention, for producing aromatic alcohols includes: an aqueous cleaning step of cleaning, with water, a composition containing aromatic peroxides, an alkali metal, and an organic solvent; and a hydrogenation step of hydrogenating the aromatic peroxides contained in the composition having been cleaned with water in the aqueous cleaning step, in the presence of an alumina-supported transition metal catalyst, in a device including a filtering device for separating the alumina-supported transition metal catalyst from a reaction liquid.

The following description will discuss [1.1] an aqueous cleaning step, [1.2] a hydrogenation step, and [1.3] other steps of the method, in accordance with an embodiment of the present invention, for producing aromatic alcohols, in this order.

### Aqueous cleaning step

The method, in accordance with an embodiment of the present invention, for producing aromatic alcohols includes an aqueous cleaning step of cleaning with water a composition containing aromatic peroxides, an alkali metal, and an organic solvent.

### (Aromatic peroxides)

In an embodiment of the present invention, the aromatic peroxides are not limited to any particular aromatic peroxides, provided that the aromatic peroxides are aromatic compounds containing a hydroperoxy group. The aromatic peroxides may be substances including a single compound, or may be substances including a mixture of two or more types of compounds. In particular, the aromatic peroxides are more preferably aromatic compounds in which at least one hydrocarbon group containing a hydroperoxy group is attached to a carbon atom of an aromatic ring. At least one hydrocarbon group containing a hydroperoxy group is needed to be attached to a carbon atom of an aromatic ring. For example, one, two, three, four, five, or six hydrocarbon groups may be attached to one or more carbon atoms of an aromatic ring(s). The hydrocarbon group may be a saturated or unsaturated hydrocarbon group. The hydrocarbon group is more preferably a saturated hydrocarbon group. The hydrocarbon group may be a linear, branched, or cyclic hydrocarbon group. The number of carbon atoms contained in the hydrocarbon group is preferably 1 to 8, more preferably 2 to 8, even more preferably 2 to 6, and particularly preferably 2 to 5. The hydrocarbon group is particularly preferably an alkyl group containing 2 to 5 carbon atoms. The aromatic compound is not limited to any particular type. Examples of the aromatic ring of the aromatic compound can include a benzene ring, a naphthalene ring, and an anthracene ring. Any carbon atom of the hydrocarbon group containing a hydroperoxy group may be attached to a carbon atom of the aromatic ring.

The aromatic compound may or may not contain another substituent. Examples of the other substituent can include a hydrocarbon group containing a hydroxy group, a hydrocarbon group containing a halogen, a hydrocarbon group containing a nitro group, a hydrocarbon group containing an amino group, a hydrocarbon group containing a carbonyl group, and a hydrocarbon group containing an alkoxy group. One type or two or more types of the other substituents can be attached to one or more carbon atoms of an aromatic ring(s) of the aromatic compound above. The number of the other substituents that can be attached to the carbon atoms of the aromatic ring(s) of the aromatic compound is not limited to any particular number. The other substituents may be of one type or two or more types, and the number thereof may be one, two, three, four, or five.

In a case where the aromatic compound contains, as the other substituent, a hydrocarbon group containing a hydroxy group, the hydrocarbon group may be a saturated or unsaturated hydrocarbon group. The hydrocarbon group is more preferably a saturated hydrocarbon group. The hydrocarbon group may be a linear, branched, or cyclic hydrocarbon group. The number of carbon atoms contained in the hydrocarbon group is preferably 1 to 8, more preferably 2 to 8, even more preferably 2 to 6, and particularly preferably 2 to 5. The hydrocarbon group is particularly preferably an alkyl group containing 2 to 5 carbon atoms. Any carbon atom of the hydrocarbon group containing a hydroxy group may be attached to a carbon atom of the aromatic ring.

In an embodiment of the present invention, a more preferable example of the aromatic peroxides can be, for example, (hydroperoxyisopropyl)(hydroxyisopropyl) benzene, bis(hydroperoxyisopropyl)benzene, or a mixture thereof. These aromatic peroxides may be any of an ortho-isomer, a meta-isomer, and a para-isomer, or may be a mixture thereof.

A more preferable example of the aromatic peroxides can be 1-(hydroperoxyisopropyl)-3-(hydroxyisopropyl)benzene, 1,3-bis(hydroperoxyisopropyl)benzene, or a mixture thereof.

In an embodiment of the present invention, a more preferable example of the aromatic peroxides can be aromatic peroxides containing, as a main component, (hydroperoxyisopropyl)(hydroxyisopropyl)benzene. As used herein, the expression "as a main component" refers to being contained in an amount, relative to a total amount of the aromatic peroxides, of not less than 50 weight%, more preferably of not less than 55 weight%, even more preferably of not less than 60 weight%, and particularly preferably of not less than 65 weight%.

### (Alkali metal)

In an embodiment of the present invention, the alkali metal is at least one selected from the group consisting of lithium, sodium, potassium, rubidium, cesium, and francium.

### (Organic solvent)

In an embodiment of the present invention, the organic solvent is not limited to any particular type. The organic solvent is more preferably a solvent capable of dissolving the aromatic peroxides above. Examples of such an organic solvent can include ketones containing 4 to 10 carbon atoms, ethers containing 4 to 10 carbon atoms, and alcohols containing 4 to 8 carbon atoms. One of these organic solvents can be used alone, or two or more of these organic solvents can be used in combination. In particular, the organic solvent is particularly preferably methyl isobutyl ketone.

### (Composition)

In an embodiment of the present invention, a composition containing the aromatic peroxides, the alkali metal, and the organic solvent is a raw material liquid for producing aromatic alcohols through a hydrogenation step, which will be described later.

The composition preferably contains the aromatic peroxides in a concentration of not less than 0.1 weight% and not more than 50 weight%, from the viewpoint of a hydrogenation reaction. The concentration of the aromatic peroxides is preferably not less than 0.1 weight% because concentrations in such a range eliminate the need for an excessively large reactor. The concentration of the aromatic peroxides is preferably not more than 50 weight% in terms of reactivity because the aromatic peroxides are sufficiently dissolved when contained in concentrations in such a range. The composition contains the aromatic peroxides in a concentration more preferably of not less than 1 weight%, and even more preferably of not less than 2 weight%. The composition contains the aromatic peroxides in a concentration more preferably of not more than 45 weight%, and even more preferably of not more than 40 weight%.

In an embodiment of the present invention, in a case where the aromatic peroxides above are, for example, aromatic peroxides containing, as a main component, 1-(hydroperoxyisopropyl)-3-(hydroxyisopropyl)benzene, a reaction liquid obtained by, for example, a method described in the chapter "(Aromatic peroxides production step)", which will be described later, can be used as the composition.

### (Aqueous cleaning)

In an embodiment of the present invention, an aqueous cleaning method used in an aqueous cleaning step is not limited to any particular method. The aqueous cleaning may be operated by a batch method or a continuous method. For example, a device including a mixing device and an oil-water separation device can be used in the aqueous cleaning. For example, a tank including a stirrer, a pipe carrying the composition and including a stirrer, or the like can be used as the mixing device. In a case where the device including a mixing device and an oil-water separation device is used in this step, the composition and water may be mixed together through stirring in the mixing device, an aqueous phase may be then separated from an oil phase in the oil-water separation device, and the aqueous phase may be then extracted from the oil-water separation device.

Alternatively, the aqueous cleaning may be carried out by a continuous method of supplying water to a pipe that carries the composition and that does not include a stirrer to carry out aqueous cleaning in the pipe. Also in a case where the aqueous cleaning is carried out by the above-described continuous method, a mixture of the composition and the water having been mixed together in the pipe may undergo separation of an aqueous phase from an oil phase in, for example, an oil-water separation device, and the aqueous phase may be then extracted from the oil-water separation device.

The water is used in a weight ratio thereof to the composition preferably of not less than 0.001, and more preferably of not less than 0.01, from the viewpoint of efficiently removing the alkali metal. The water is used in a weight ratio thereof to the composition preferably of not more than 5, more preferably of not more than 3, and particularly preferably of not more than 0.02, from the viewpoint of drainage volume.

The water has a temperature preferably of not more than 80°C, and more preferably of not more than 70°C, from the viewpoint of energy conservation. The temperature of the water is preferably of not less than 5°C, and more preferably of not less than 10°C, from the viewpoint of operability.

The aqueous cleaning is carried out preferably for 1 second to 600 minutes, and more preferably for 10 seconds to 300 minutes.

The aqueous cleaning may be carried out one time or more than one time. For example, the aqueous cleaning may be repeated two or three times.

In an embodiment of the present invention, the composition having been cleaned with water in the aqueous cleaning step above contains the alkali metal in a concentration preferably of not less than 0.0005 mmol/kg and not more than 0.18 mmol/kg, more preferably of not less than 0.001 mmol/kg and not more than 0.18 mmol/kg, even more preferably of not less than 0.005 mmol/kg and not more than 0.18 mmol/kg, particularly preferably of not less than 0.01 mmol/kg and not more than 0.18 mmol/kg, and most preferably of not less than 0.01 mmol/kg and not more than 0.05 mmol/kg. When the composition having been cleaned with water in the aqueous cleaning step contains the alkali metal in the above concentration ranges, effects thereby produced are that, when a hydrogenation reaction is carried out in the presence of an alumina-supported transition metal catalyst in a device including a filtering device for separating the alumina-supported transition metal catalyst from a reaction liquid, it is possible to reduce clogging of the filtering device and thereby improve the production efficiency.

### Hydrogenation step

A method, in accordance with an embodiment of the present invention, for producing aromatic alcohols includes a hydrogenation step of hydrogenating the aromatic peroxides contained in the composition having been cleaned with water in the aqueous cleaning step, in the presence of an alumina-supported transition metal catalyst, in a device including a filtering device for separating the alumina-supported transition metal catalyst and a reaction liquid.

The hydrogenation step is a step of hydrogenating the aromatic peroxides contained in the composition having been cleaned with water to obtain a reaction liquid containing aromatic alcohols.

In an embodiment of the present invention, the hydrogenation is carried out in a device including a filtering device for separating an alumina-supported transition metal catalyst from a reaction liquid. It is therefore possible to continuously obtain a reaction liquid containing aromatic alcohols by separating the obtained reaction liquid from the alumina-supported transition metal catalyst. The filtering device is not limited to any particular form. The form of the filtering device is, for example, a filter, and more preferably a metal filter.

The filtering device may be provided inside or outside the reaction container of the device, and is more preferably provided inside the reaction container. The filtering device being provided inside the reaction container eliminates the need to put back, into the reaction container, the alumina-supported transition metal catalyst having been separated. This prevents the structure of the device from being complex, and is thus preferable.

In an embodiment of the present invention, the hydrogenation is carried out in the presence of an alumina-supported transition metal catalyst. The alumina-supported transition metal catalyst may be any catalyst that is a transition metal catalyst supported on alumina. The transition metal catalyst is not limited to any particular catalyst. As the transition metal catalyst, noble metals belonging to group VIII of the periodic table can be suitably used. Examples of such a transition metal catalyst can include palladium, nickel, and platinum. One of these metals may be used alone, or two or more of these metals can be used in combination. In other words, the transition metal catalyst can be at least one selected from palladium, nickel, and platinum. In particular, a palladium catalyst can be particularly preferably used from the viewpoint of activity and selectivity. The concentration of the transition metal catalyst supported on an alumina support is typically 0.01 weight% to 20 weight%, relative to the weight of the alumina support.

In an embodiment of the present invention, hydrogen used in the hydrogenation is not required to be pure, and may contain nitrogen, carbon dioxide, methane, helium, argon, or other inert gas. The hydrogenation is carried out under a reaction pressure typically of 0 MPa to 10 MPa (gauge pressure). Further, the hydrogenation is carried out at a reaction temperature typically of 20°C to 200°C, and for a reaction time typically of 1 minute to 300 minutes. The hydrogenation can be carried out by a reaction method such as a fixed-bed flow reaction in a liquid phase or a stirring tank slurry reaction. As the reaction method, a stirring tank slurry reaction with the use of a powder catalyst is suitable in terms of activity, selectivity, catalyst life, etc. As the form of the alumina-supported transition metal catalyst, a pellet catalyst molded by tableting, extruding, etc. can be used in a case of the fixed-bed flow reaction in a liquid phase, and a powder catalyst or the above-described pellet catalyst can be used in a case of the stirring tank slurry reaction. In the case of the stirring tank slurry reaction, the concentration of the catalyst is in a range typically of 0.01 weight% to 10 weight%.

In an embodiment of the present invention, when the hydrogenation is carried out by the stirring tank slurry reaction, the effect of the present invention is particularly significantly produced.

In an embodiment of the present invention, aromatic alcohols obtained in the hydrogenation step are not limited to any particular aromatic alcohols, provided that the aromatic alcohols are aromatic compounds containing a hydroxy group via a carbon atom that is not a carbon atom of an aromatic ring(s). The aromatic alcohols may be substances including a single compound, or may be substances including a mixture of two or more types of compounds. In particular, the aromatic alcohols are more preferably aromatic compounds in which at least one hydrocarbon group containing a hydroxy group is attached to a carbon atom of the aromatic ring(s). At least one hydrocarbon group containing a hydroxy group is needed to be attached to a carbon atom of the aromatic ring(s). For example, one, two, three, four, five, or six hydrocarbon groups may be attached to one or more carbon atoms of the aromatic rings(s). Two hydrocarbon groups, each containing a hydroxy group, are more preferably attached to carbon atoms of the aromatic ring(s). The hydrocarbon group may be a saturated or unsaturated hydrocarbon group. The hydrocarbon group is more preferably a saturated hydrocarbon group. The hydrocarbon group may be a linear, branched, or cyclic hydrocarbon group. The hydrocarbon group may need to contain 1 to 8 carbon atoms, more preferably contains 2 to 8 carbon atoms, and even more preferably contains 2 to 6 carbon atoms. The hydrocarbon group is particularly preferably an alkyl group containing 2 to 5 carbon atoms. The aromatic compound is not limited to any particular type. Examples of an aromatic ring of the aromatic compound can include a benzene ring, a naphthalene ring, and an anthracene ring. Any carbon atom of the hydrocarbon group containing a hydroxy group may be attached to a carbon atom of the aromatic ring(s).

In an embodiment of the present invention, more preferable examples of the aromatic alcohols can include aromatic alcohols that contain, for example, bis(2-hydroxy-2-propyl)benzene as a main component. The bis(2-hydroxy-2-propyl)benzene may be any of an ortho-isomer, a meta-isomer, and a para-isomer, or may be a mixture thereof.

In particular, the aromatic alcohols are more preferably aromatic alcohols containing 1,3-bis(2-hydroxy-2-propyl)benzene as a main component.

### Other steps

The method, in accordance with an embodiment of the present invention, for producing aromatic alcohols can further include other steps. The other steps can include an aromatic peroxides production step of producing the aromatic peroxides contained in the composition to be subjected to the aqueous cleaning step, and a post-treatment step of purifying aromatic alcohols obtained in the hydrogenation step.

### (Aromatic peroxides production step)

In an embodiment of the present invention, an aromatic peroxides production step is not limited to any particular step. The following description will discuss, by way of example, a case where the aromatic peroxides contain, for example, 1-(hydroperoxyisopropyl)-3-(hydroxyisopropyl)benzene as a main component. However, the aromatic peroxides production step is not limited to this.

For example, a reaction liquid obtained in the aromatic peroxides production step described below can be suitably used as the composition.

Examples of the reaction liquid obtained in the aromatic peroxides production step can include a liquid (i) and a liquid (ii) obtained as follows: Meta-diisopropylbenzene undergoes an oxidation reaction to provide an oxidation reaction liquid containing 1,3-bis(hydroperoxyisopropyl)benzene. Further, in a step of separating the 1,3-bis(hydroperoxyisopropyl)benzene from the oxidation reaction liquid by organic solvent extraction or the like and then obtaining resorcin by subjecting the 1,3-bis(hydroperoxyisopropyl)benzene to a decomposition reaction, a liquid (i) remaining after the separation of the 1,3-bis(hydroperoxyisopropyl)benzene from the oxidation reaction liquid is obtained. A liquid (ii) is obtained by removing a part of the organic solvent from the liquid (i) by a distilling operation or the like and then concentrating the liquid (i). The following description will discuss each of the steps.

Oxidation step: a step of subjecting meta-diisopropylbenzene to an oxidation reaction in the air to obtain an oxidation reaction liquid containing 1,3-bis(hydroperoxyisopropyl)benzene and 1-(hydroperoxyisopropyl)-3-(hydroxyisopropyl)benzene.

The oxidation step is a step of oxidizing an oxidation raw material liquid containing diisopropylbenzene with oxygen or air. The oxidation raw material liquid typically contains meta-diisopropylbenzene, which is a main raw material, in a concentration of 1 weight% to 100 weight%. Typical reaction conditions can include a temperature of 70°C to 110°C, a pressure of 0 MPa to 1 MPa (gauge pressure), and a residence time of 0 hours to 50 hours. Examples of a device usable in the oxidation step can include a flow-type reaction tank, a batch-type reaction tank, and a tube reactor. Examples of the oxidation reaction liquid obtained in the oxidation step can include a liquid containing 1,3-bis(hydroperoxyisopropyl)benzene, 1-(hydroperoxyisopropyl)-3-(hydroxyisopropyl)benzene, and meta-diisopropylbenzene.

Separation step: a step of separating the oxidation reaction liquid obtained in the oxidation step into a liquid containing 1,3-bis(hydroperoxyisopropyl)benzene and a liquid containing 1-(hydroperoxyisopropyl)-3-(hydroxyisopropyl)benzene by subjecting the oxidation reaction liquid to an extracting operation through an aqueous sodium hydroxide solution and an extracting operation through an organic solvent.

The separation step is a step of separating the oxidation reaction liquid obtained in the oxidation step into a liquid containing 1,3-bis(hydroperoxyisopropyl)benzene and a liquid containing 1-(hydroperoxyisopropyl)-3-(hydroxyisopropyl)benzene by subjecting the oxidation reaction liquid to an extracting operation through an aqueous sodium hydroxide solution and an extracting operation through an organic solvent.

The oxidation reaction liquid obtained in the oxidation step is first subjected to an extracting operation through an aqueous sodium hydroxide solution. The oxidation reaction liquid contains diisopropylbenzene that remains unreacted, 1,3-bis(hydroperoxyisopropyl)benzene, 1-(hydroperoxyisopropyl)-3-(hydroxyisopropyl)benzene, and other by-products. Among these substances, 1,3-bis(hydroperoxyisopropyl)benzene and 1-(hydrope roxyisopropyl)-3-(hydroxyisopropyl)benzene are extracted in the aqueous sodium hydroxide solution. In this extraction, appropriate amounts of an aqueous sodium hydroxide solution and/or meta-diisopropylbenzene may be additionally added to the oxidation reaction liquid for increasing oil-water separation performance and extraction efficiency.

The reaction temperature of the extraction is typically in a range of 0°C to 100°C. The concentration of the aqueous sodium hydroxide solution used when 1,3-bis(hydroperoxyisopropyl)benzene and 1-(hydroperoxyisopropyl)-3-(hydroxyisopropyl)benzene are extracted is preferably 2 weight% to 30 weight%, and more preferably 4 weight% to 15 weight%. The concentration being too low can cause a worsening of extraction efficiency. On the other hand, the concentration being too high can lead to an increase in alkali usage and deterioration of active components including bisl,3-bis(hydroperoxyisopropyl)benzene and 1-(hydroperoxyisopropyl)-3-(hydroxyisopropyl)benzene.

The aqueous sodium hydroxide solution containing 1,3-bis(hydroperoxyisopropyl)benzene and 1-(hydrope roxyisopropyl)-3-(hydroxyisopropyl)benzene that are obtained by the extracting operation through an aqueous sodium hydroxide solution is then subjected to an extracting operation through an organic solvent. This enables individual separation of 1,3-bis(hydroperoxyisopropyl)benzene and 1-(hydroperoxyisopropyl)-3-(hydroxyisopropyl)benzene.

The organic solvent used in the separation step is preferably ketones containing 4 to 10 carbon atoms, ethers containing 4 to 10 carbon atoms, or alcohols containing 4 to 8 carbon atoms. From the viewpoint of extraction efficiency and solvent recovery, the organic solvent used in the separation step is most preferably methyl isobutyl ketone. As the solvent, one solvent may be used alone, or a mixture of two or more types of solvents may be used.

### (Post-treatment step)

In an embodiment of the present invention, the post-treatment step is a step of purifying aromatic alcohols through the use of operations, including crystallization, filtration, and drying, performed on the reaction liquid obtained in the hydrogenation step and containing the aromatic alcohols. A concentrating operation for removing a part of the organic solvent in distillation may be carried out prior to the crystallization operation.

The crystallization operation is an operation of causing aromatic alcohols to be selectively deposited. For example, while the reaction liquid containing the aromatic alcohols or a condensed liquid thereof are being cooled in a crystallization tank while being stirred, aromatic alcohols are selectively deposited.

The filtration operation is an operation of separating crystals of the aromatic alcohols from the organic solvent, and includes depressurization, pressurization, and centrifugation. The filtration operation is preferably centrifugal filtration from the viewpoint of operability.

The drying operation is an operation of distilling the organic solvent and water adhering to the crystals of the aromatic alcohols obtained in the filtration operation to obtain aromatic alcohols as a product. The drying can be carried out under a pressure of 5 KPa to 100 KPa and at a temperature of 20°C to 100°C.

The present invention is not limited to the embodiments, but can be altered by a skilled person in the art within the scope of the claims. The present invention also encompasses, in its technical scope, any embodiment derived by combining technical means disclosed in differing embodiments.

The present invention includes the inventions indicated in the following items [1] to [6].
[1] A method for producing aromatic alcohols including: an aqueous cleaning step of cleaning, with water, a composition containing aromatic peroxides, an alkali metal, and an organic solvent; and a hydrogenation step of hydrogenating the aromatic peroxides contained in the composition having been cleaned with water in the aqueous cleaning step, in the presence of an alumina-supported transition metal catalyst, in a device including a filtering device for separating the alumina-supported transition metal catalyst from a reaction liquid.
[2] The method described in [1], wherein the composition having been cleaned with water in the aqueous cleaning step contains the alkali metal in a concentration of not less than 0.0005 mmol/kg and not more than 0.18 mmol/kg.
[3] The method described in [1] or [2], wherein the aromatic peroxides are (hydroperoxyisopropyl)(hydroxyisopropyl)benzene, bis(hydroperoxyisopropyl)benzene, or a mixture thereof, and the aromatic alcohols obtained through the hydrogenation step contain, as a main component, bis(2-hydroxy-2-propyl) benzene.
[4] The method described in any one of claims [1] to [3], wherein the aromatic peroxides are 1-(hydroperoxyisopropyl)-3-(hydroxyisopropyl)benzene, and the aromatic alcohols obtained through the hydrogenation step contain, as a main component, 1,3-bis(2-hydroxy-2-propyl)benzene.
[5] The method described in any one of claims [1] to [4], wherein the alkali metal is sodium.
[6] The method described in any one of [1] to [5], wherein the transition metal is at least one selected from palladium, nickel, and platinum.

### Examples

The present invention will be described below in more detail through Examples and Comparative Examples. However, the present invention is not limited to the Examples below.

### [Example 1]

An oxidation reaction of meta-diisopropylbenzene in the air was carried out to provide an oxidized oil containing 1-(hydroperoxyisopropyl)-3-(hydroxyisopropyl)benzene (which, hereinafter, can be referred to as CHPO) and 1,3-bis(hydroperoxyisopropyl)benzene (which, hereinafter, can be referred to as DHPO). The resultant oxidized oil underwent an extracting operation through 7 weight% aqueous sodium hydroxide solution, and then underwent an extracting operation through methyl isobutyl ketone so that a methyl isobutyl ketone solution (solution I, a composition containing aromatic peroxides, an alkali metal, and an organic solvent) containing CHPO and DHPO was obtained.

An aqueous cleaning of the solution I thereby obtained was carried out by adding, to the solution I, water in a weight ratio thereof to the solution I of 0.018, and mixing the solution I and the water in a pipe. After the aqueous cleaning, the solution I to which the water had been added was caused to stand. An aqueous phase separated in the solution was then moved apart so that a methyl isobutyl ketone solution (solution II-1) was obtained. The solution II-1 contained 0.040 mmol/kg sodium, 5.4 weight% CHPO, and 0.2 weight% DHPO.

Hydrogen and the solution II-1 were continuously supplied, in such a manner that the molar ratio of the hydrogen to CHPO of the solution II-1 was 3.7, to a reactor which includes: a stirrer; and a metal filter for separating an alumina-supported transition metal catalyst from a reaction liquid to extract the reaction liquid and in which an alumina-supported palladium catalyst was present as a catalyst in such a manner that palladium of which the weight ratio to alumina was 1 weight% is supported so that the palladium catalyst concentration in the reactor was 2 kg/m³. Under conditions inside the reactor where a liquid temperature was 98°C and a pressure was 700 kPa (gauge pressure), CHPO and DHPO in the solution II and hydrogen were caused to react together. A liquid thereby obtained was passed through the metal filter to be separated from the alumina-supported transition metal catalyst, and then continuously extracted to the outside of the reactor, so that a reaction liquid containing 1,3-bis(2-hydroxy-2-propyl)benzene and methyl isobutyl ketone was obtained. In this filtration, an increase in the differential pressure of the filter was 1.0 kPa/day.

From the reaction liquid thereby obtained, methyl isobutyl ketone was partially evaporated and removed in a concentrating operation, so that a condensed liquid was obtained. From the condensed liquid thereby obtained, white crystals of 1,3-bis(2-hydroxy-2-propyl)benzene were obtained through crystallization, separation, and subsequent drying operations.

### [Example 2]

Operations the same as those in Example 1 were carried out, except that the amount of water relative to the solution I used in the aqueous cleaning was changed from 0.018 to 0.014 by weight ratio, to provide a solution 11-2. The solution 11-2 contained 0.048 mmol/kg sodium, 5.4 weight% CHPO, and 0.2 weight% DHPO.

The solution 11-2 thereby obtained underwent operations the same as those in Example 1, so that white crystals of 1,3-bis(2-hydroxy-2-propyl)benzene were obtained. An increase in the differential pressure of the filter was 0.8 kPa/day.

### [Example 3]

Operations the same as those in Example 1 were carried out, except that the amount of water relative to the solution I used in the aqueous cleaning was changed from 0.018 to 0.011 by weight ratio, to provide a solution 11-3. The solution 11-3 contained 0.061 mmol/kg sodium, 5.3 weight% CHPO, and 0.2 weight% DHPO.

Operations the same as those in Example 1 were carried out, except that hydrogen and the solution 11-3 thereby obtained were continuously supplied in such a manner that the molar ratio of the hydrogen to CHPO of the solution 11-3 was 3.8 and the palladium catalyst concentration in the reactor was changed to 3 kg/m³, to provide white crystals of 1,3-bis(2-hydroxy-2-propyl)benzene. An increase in the differential pressure of the filter was 1.3 kPa/day.

### [Example 5]

Operations the same as those in Example 1 were carried out, except that the amount of water relative to the solution I used in the aqueous cleaning was changed from 0.018 to 0.009 by weight ratio, to provide a solution II-4. The solution 11-4 contained 0.10 mmol/kg sodium, 5.5 weight% CHPO, and 0.3 weight% DHPO.

Operations the same as those in Example 1 were carried out, except that hydrogen and the solution 11-4 thereby obtained were continuously supplied in such a manner that the molar ratio of the hydrogen to CHPO of the solution 11-4 was 3.6 and the palladium catalyst concentration in the reactor was changed to 4 kg/m³, to provide white crystals of 1,3-bis(2-hydroxy-2-propyl)benzene. An increase in the differential pressure of the filter was 1.5 kPa/day.

### [Comparative Example 1]

Operations the same as those in Example 1 were carried out, except that an aqueous cleaning was not carried out, to provide a solution II-5. The solution II-5 contained 0.19 mmol/kg sodium, 5.8 weight% CHPO, and 0.2 weight% DHPO.

Operations the same as those in Example 1 were carried out, except that hydrogen and the solution 11-5 thereby obtained were continuously supplied in such a manner that the molar ratio of the hydrogen to CHPO of the solution 11-5 was 3.5, to provide white crystals of 1,3-bis(2-hydroxy-2-propyl)benzene. An increase in the differential pressure of the filter was 6.6 kPa/day.

Listed in Table 1 below are weight ratios of the cleaning water to the solution I, Na concentrations, CHPO concentrations, and DHPO concentrations in the solution II (solution II-1 to 11-5), reaction conditions for a hydrogenation reaction, and increases in differential pressures, in relation to Examples 1 to 4 and Comparative Example 1.

**[Table 1]**

| | Cleaning water/ solution I | Solution II | | | Reaction conditions | | Increase in differential pressure |
|---|---|---|---|---|---|---|---|
| | weight ratio | Na concentration | CHPO | DHPO | Catalyst concentration | H₂/ CHPO | kPa/day |
| | | mmol/kg | weight% | weight% | kg/m³ | molar ratio | |
| Example 1 | 0.018 | 0.040 | 5.4 | 0.2 | 2 | 3.7 | 1 |
| Example 2 | 0.014 | 0.048 | 5.4 | 0.2 | 2 | 3.7 | 0.8 |
| Example 3 | 0.011 | 0.061 | 5.3 | 0.2 | 3 | 3.8 | 1.3 |
| Example 4 | 0.009 | 0.10 | 5.5 | 0.3 | 4 | 3.6 | 1.5 |
| Comparative Example 1 | 0.000 | 0.19 | 5.8 | 0.2 | 2 | 3.5 | 6.6 |

### (Conclusions)

As shown in Table 1, the solutions II (II-1 to 11-4), which are raw material liquids in hydrogenation, obtained by cleaning the solution I with water in the production methods of Examples 1 to 4 that include a step of cleaning the solution I with water contain an alkali metal in lower concentrations than the solution II obtained by the production method of Comparative Example 1 that does not include the step of cleaning the solution I with water. It has also been shown that a lower alkali metal concentration in the solution II leads to a smaller amount of increase in the differential pressure. It has also been shown that the increase in a differential pressure is significantly smaller in Examples 1 to 4 in which the solutions II (II-1 to 11-4), which are raw material liquids in hydrogenation, obtained by cleaning the solution I with water are used as raw material liquids than in Comparative Example 1 that does not include the step of cleaning the solution I with water. This indicates that a lower alkali metal concentration in the solution II leads to a reduction in clogging of the metal filter.

### Industrial Applicability

The production method in accordance with an embodiment of the present invention enables efficient production of aromatic alcohols including 1,3-di-(2-hydroxy-2-propyl)-benzene. The present invention is therefore useful in fields in which such aromatic alcohols are used.

## Claims

1. A method for producing aromatic alcohols, the method comprising:
an aqueous cleaning step of cleaning, with water, a composition containing aromatic peroxides, an alkali metal, and an organic solvent; and
a hydrogenation step of hydrogenating the aromatic peroxides contained in the composition having been cleaned with water in the aqueous cleaning step, in the presence of an alumina-supported transition metal catalyst, in a device including a filtering device for separating the alumina-supported transition metal catalyst from a reaction liquid.

2. The method according to claim 1, wherein the composition having been cleaned with water in the aqueous cleaning step contains the alkali metal in a concentration of not less than 0.0005 mmol/kg and not more than 0.18 mmol/kg.

3. The method according to claim 1 or 2, wherein the aromatic peroxides are (hydroperoxyisopropyl)(hydroxyisopropyl)benzene, bis(hydroperoxyisopropyl)benzene, or a mixture thereof, and the aromatic alcohols obtained through the hydrogenation step contain, as a main component, bis(2-hydroxy-2-propyl) benzene.

4. The method according to any one of claims 1 to 3, wherein the aromatic peroxides are 1-(hydroperoxyisopropyl)-3-(hydroxyisopropyl)benzene, and the aromatic alcohols obtained through the hydrogenation step contain, as a main component, 1,3-bis(2-hydroxy-2-propyl)benzene.

5. The method according to any one of claims 1 to 4, wherein the alkali metal is sodium.

6. The method according to any one of claims 1 to 5, wherein the transition metal is at least one selected from palladium, nickel, and platinum.
